# EUROPÄISCHE PATENTSCHRIFT

(11) **EP 1 382 383 B1**
(45) Veröffentlichungstag und Bekanntmachung des Hinweises auf die Patenterteilung: **17.08.2005**
(21) Anmeldenummer: 03015575.8
(22) Anmeldetag: 11.07.2003
(51) Int. Cl.: B01J 8/04, B01J 23/50, B01J 23/04, B01J 27/18, B01J 23/00, C07C 47/21, C07D 303/04

(54) **Strukturierte Katalysator-Schüttung**
Ordered catalyst packing
Garnissage ordonné de catalyseurs

(30) Priorität: 15.07.2002 DE 10231976
(43) Veröffentlichungstag der Anmeldung: 21.01.2004
(73) Patentinhaber: BASF AKTIENGESELLSCHAFT, 67056 Ludwigshafen (DE)
(72) Erfinder: Hibst, Hartmut Prof.Dr., 69198 Schriesheim (DE); Storck, Sebastian Dr., 68167 Mannheim (DE); Demuth, Dirk Dr., 69226 Nussloch (DE); Stichert, Wolfram Dr., 69124 Heidelberg (DE); Klein, Jens, 69124 Heidelberg (DE); Schunk, Stephan A. Dr., 69115 Heidelberg (DE); Sundermann, Andreas, 69120 Heidelberg (DE)
(74) Vertreter: Isenbruck, Günter, Dr.

(56) Entgegenhaltungen:
- EP-A- 0 326 392
- DE-A- 4 400 837
- DE-A- 10 137 534
- DE-A- 10 230 221
- US-A- 4 925 986
- US-A- 4 925 987
- US-A- 4 942 263

## Beschreibung

Die vorliegende Erfindung betrifft eine neuartige strukturierte Katalysator-Schüttung, wobei die strukturierte Katalysator-Schüttung optional mindestens eine Teil-Schüttung enthält, die mindestens eine katalytisch aktive Mischung von Oxiden der Haupt- und Nebengruppenmetalle enthält, sowie weiterhin mindestens eine katalytisch aktive Teil-Schüttung umfassend mindestens Silber, mindestens ein Alkalimetall sowie ein poröses Trägermaterial, sowie schließlich zwingend mindestens eine katalytisch aktive Teil-Schüttung, umfassend mindestens ein Alkalimetall-Phosphat sowie mindestens ein Schichtsilikat.

Weiterhin betrifft die vorliegende Erfindung ein Verfahren zur Herstellung von Crotonaldehyd aus C₄- Mono- oder Diolefinen unter Verwendung der besagten strukturierten Katalysator-Schüttung.

In einer Ausführungsform der vorliegenden Erfindung wird ein Verfahren für die Direktsynthese von Crotonaldehyd beansprucht, umfassend die folgenden Reaktions-Schritte:
(1) Dehydrierung von Buten zu 1,3-Butadien
(2) Epoxidation von 1,3-Butadien zu 1,2-Epoxy-3-buten (Vinyloxiran)
(3) Isomerisierung von 1,2-Epoxy-3-buten zu 2-Butenal (Crotonaldehyd)

In der vorliegenden Anmeldung ist unter "Butadien" das C₄-Diolefin 1,3 Butadien, unter "Vinyloxiran" 1,2-Epoxy-3-buten, sowie unter "Crotonaldehyd" 2-Butenal zu verstehen.

Dabei ist der Schritt (1) optional, wenn als Edukt Butadien und nicht Buten eingesetzt werden soll.

Im Folgenden ist der Stand der Technik bezüglich eines jeden einzelnen Reaktions-Schrittes sowie möglicher Kombinationen beschrieben.

Die Dehydrierung von Buten, d.h. die Reaktion (1), kann beispielsweise nach dem Dow-Verfahren unter Zusatz von Wasserdampf erfolgen [siehe z. B. K. Weissermel, H.-J. Arpe in: *Industrielle Organische Chemie*, 5. Auflage (1998), 123]. Hierbei wird eine Butadien Selektivität von etwa 90% bei einem Umsatz von etwa 50% unter Verwendung eines mit Chromoxid stabilisierten Ca-Ni-Phosphat-Katalysators bei Temperaturen von 600 bis 675°C erreicht. Ein alternatives Verfahren ist durch die Oxydehydrierung gegeben, wie sie beispielsweise von Petro-Tex unter Verwendung eines heterogenen Katalysators mit einem Ferrit der Metalle Zn, Mn oder Mg implementiert wurde. Die Zugabe von Sauerstoff bewirkt dabei nicht nur die nachträgliche Wasserstoff-Verbrennung, sondern leitet auch die Dehydrierung durch Abstraktion von Wasserstoff aus der Allyl-Stellung ein [siehe K. Weissermel, H.-J. Arpe in: *Industrielle Organische Chemie*, 5. Auflage (1998), 124].

Eine zweistufige Synthese von Crotonaldehyd aus Buten (unter Umgehung der Bildung von Vinyloxiran) ist in mehreren Arbeiten von J. Haber und Kollegen beschrieben [siehe beispielsweise J. Haber und T. Wiltowski, *Bull. Acad. Pol. Sci., Ser. Sci. Chim.* **29** (1983), 563; J. Haber und M. Witko, *Catal. Letters* **9** (1991) 297]. Der dabei eingesetzte Katalysator basiert auf Kupfer-Molybdaten (CuMoO₄) und ändert seine Zusammensetzung im Verlauf der Reaktion. Wird reines Buten über den Katalysator geleitet, so bilden sich monovalente Kupfer-Molybdate, wobei "Cu₂Mo₃O₁₀" die Isomerisierung von Buten zu Butadien fördert, während "Cu₆Mo₄O₁₅" bei Selektivitäten von bis zu 70% zum Einschub von Sauerstoff und somit zur Bildung von Crotonaldehyd führt. Diese Reaktionen werden allerdings alle im instationären Betrieb durchgeführt, d.h. unter Verwendung von Gas-Pulsen, die in einen inerten Gas-Strom eingeführt werden. So werden beispielsweise mittels einer Spritze Edukt-Schübe im Milliliter-Bereich in einen He-Strom injiziert. Dieser Strom wird dann über einen Katalysator geleitet. Über die Durchführung einer solchen Reaktion im für die Praxis wichtigen stationären Betrieb wird in den o.a. Druckschriften allerdings keine Lehre erteilt.

Morselli et al. erreichen bei 25 % Umsatz eine 80 Mol-% Selektivität für die Umsetzung von Buten zu Crotonaldehyd (welches neben Furan und Maleinsäure als Nebenprodukt anfällt), wiederum unter instationären Versuchsbedingungen, d.h. unter Verwendung eines gepulsten Mikro-Reaktors [siehe Morselli et al., *J.Catal.* **75** (1982), 112]. Dabei verwenden die Autoren einen Vanadium-Phosphor-Oxid-Katalysator. Das Feed-Gas beinhaltet keinen Sauerstoff. Auch hier ist als Nachteil zu nennen, dass instationäre Bedingungen für großtechnische Anwendungen nicht geeignet sind.

Die besten Selektivtäten für die Umsetzung von n-Butenen zu Crotonaldehyd unter stationären Bedingungen wurden von Popova et al. publiziert [N.L Popova und F.A.Mil'man, *Kinetics & Catalysis* **6** (1965) 856; Übersetzung der russischen Ausgabe]. In dieser Studie werden Cu-Oxid/SiOₓ-Katalysatoren verwendet. Die dort angegebenen Selektivitäten von 16 Mol-%, erzielt allerdings bei sehr geringen Umsätzen, sind die besten Selektivitäten nach dem Stand der Technik, wie sie bei Verwendung von Sauerstoff im Eduktgas (feed-Gas) auftreten. Sowohl die Selektivität als auch insbesondere der niedrige Umsatz bedingen allerdings, dass das in dieser Druckschrift beschriebene Verfahren nicht für den (groß)industriellen Einsatz geeignet ist.

Bezüglich der Reaktion (2), d.h. der Umsetzung von Butadien zu Vinyloxiran ist die EP-A 0 326 392 (*Selective epoxidation of olefins,* Eastman Kodak) zu nennen. Dort wird u.a. für die Umsetzung von 1,3-Butadien zu Vinyloxiran der Einsatz von silberhaltigen Katalysatoren bei Verwendung eines Sauerstoff-enthaltenden Gases in einem Temperaturbereich zwischen 75 und 325 °C bei Umsätzen zwischen 0,1 und 75 % beschrieben. Bezüglich einer Weiterreaktion des Vinyloxirans zu Crotonaldehyd wird keine Lehre erteilt, sondern es wird vielmehr explizit nur auf das Bereitstellen von katalytischen Verfahren zur Epoxidierung von Olefinen, die längerkettig sind als Ethylen, abgestellt.

Bezüglich der direkten Umsetzung von Butadien zu Crotonaldehyd, d.h. der Kombination der Reaktionen (2) und (3), ist die US 4 942 263 zu nennen. Diese Druckschrift von Eastman Kodak beansprucht ein Verfahren zur direkten Herstellung von Crotonaldehyd aus 1,3-Butadien und Sauerstoff. Als Katalysator wird metallisches Silber auf einem Substrat mit einer Oberfläche von mindestens 50 m²/g. beansprucht. Als Nachteil dieses Verfahrens nach dem Stand der Technik ist insbesondere zu nennen, dass nur äußerst geringe Umsätze von weniger als 1% erreicht werden (siehe vergleichende Diskussion unten).

Schließlich ist noch der Stand der Technik bezüglich der Reaktion (3), d.h. der Isomerisierung von 1,2-epoxy-3-Buten zu Crotonaldehyd zusammenzufassen. In diesem Zusammenhang hat Eastman Kodak gleichfalls zwei Patente veröffentlicht: 1.) US 4 925 986. Als Katalysator beansprucht werden hier einige Alkali-, Erdalkali- und Übergangsmetallhalogenide; 2.) US 4 925 987. Als Katalysator werden in diesem Patent binäre und gemischte Metalloxide der Gruppen Ib und IIb beansprucht. Die Verwendung von Li₃PO₄ ist in diesen beiden Schriften nicht offenbart. Die Umsetzung von Vinyloxiran zu Crotonaldehyd über Li₃PO₄ in einer flüssigen Lösungsmittel-Phase (Hexan) wurde von Srednev et al. [Russ. *J. Org. Chem.* **34** (1998), 968] beschrieben. Bezüglich einer entsprechenden Reaktion in der Gasphase wird jedoch keine Lehre erteilt.

Zusammenfassend offenbart der Stand der Technik also kein stationäres Verfahren zur direkten Synthese von Crotonaldehyd aus Buten oder auch aus Butadien, welches mit für großtechnische Anwendungen relevanten Umsätzen abliefe. Aufgrund der hier zusammengefassten Nachteile der Verfahrensführung über Butadien und/oder Vinyloxiran wird Crotonaldehyd bis dato großtechnisch nicht auf dieser Route hergestellt sondern beispielsweise durch Dimerisierung und Dehydratisierung von Acetaldehyd (Aldolkondensation), und zwar diskontinuierlich in der Flüssigphase [siehe z. B. K. Weissermel, H.-J. Arpe in: *Industrielle Organische Chemie,* 5. Auflage (1998), S. 204].

Somit bestand die Aufgabe der vorliegenden Erfindung darin, einen neuartige Katalysator bereitzustellen, wobei der besagte Katalysator insbesondere zur direkten Synthese von Crotonaldehyd aus Buten oder aus Butadien eingesetzt werden kann. Die erfindungsgemäße Aufgabe bestand auch darin, ein Verfahren unter Verwendung des in Rede stehenden Katalysators bereitzustellen, wobei dieser Katalysator vorzugsweise kostengünstig in einem Festbettreaktor, d.h. unter stationären Bedingungen eingesetzt werden kann und Umsätze ermöglicht, die über die Umsätze, die nach dem Stand der Technik erhalten werden, hinausgehen.

Diese Aufgabe wird dadurch gelöst, dass eine neue strukturierte Katalysator-Schüttung bereitgestellt wird, die beispielsweise in einen Festbettreaktor gegeben werden kann, wodurch ein stationärer Reaktionsablauf ermöglicht wird.

Ein Vorteil der in der vorliegenden Erfindung beanspruchten Katalysator-Schüttung liegt darin, dass sie einen Weg eröffnet, C₄ - Mono- oder Diolefine in einem einzigen Festbettreaktor kontinuierlich und in Ausbeuten größer als 1% zu Crotonaldehyd umzusetzen.

Die Verwendung der erfindungsgemäßen Katalysator-Schüttung wird beispielhaft für die Direktsynthese von Crotonaldehyd aus Buten oder Butadien dargestellt. Dies bedeutet allerdings nicht, dass das neue Material, d.h. die strukturierte Katalysator-Schüttung nicht auch für beliebige andere Reaktionen eingesetzt werden könnte.

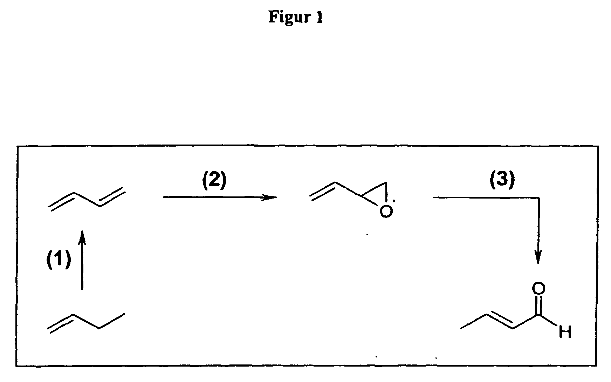

Die verschiedenen Teilreaktionen und die chemischen Formeln der Hauptprodukte bzw. Edukte der in Rede stehenden beispielhaften Reaktion sind schematisch in der Figur 1 dargestellt.

Die einzelnen Teilreaktionen bzw. Reaktionsschritte sind dabei, wie bereits oben angegeben:
(1) Dehydrierung von Buten zu 1,3-Butadien
(2) Epoxidation von 1,3-Butadien zu 1,2-Epoxy-3-buten (Vinyloxiran)
(3) Isomerisierung von 1,2-Epoxy-3-buten zu 2-Butenal (Crotonaldehyd)

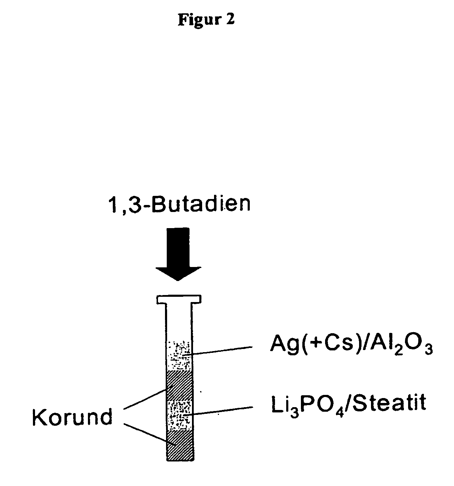

Figur 2 zeigt eine zweifache Katalysator-Schüttung, wie sie eingesetzt wird, wenn Butadien das Edukt ist, d.h. wenn der Reaktionsschritt (1) entfällt.

Die zum Reaktionsschritt (2) gehörige Teil-Schüttung (II) enthält mindestens ein katalytisch aktives Material und ist in Figur 2 schematisch als "Ag(+Cs)/Al₂O₃" dargestellt. Als katalytisch aktives Material für diese Teil-Schüttung sind prinzipiell alle Katalysatoren geeignet, welche die Teilreaktion (2) bezüglich Selektivität und/oder Umsatz befördern. In einer bevorzugten Ausführungsform enthält der Katalysator dieser Teil-Schüttung (II) mindestens Silber, mindestens ein Alkalimetall und ist auf mindestens einen porösen Träger aufgebracht. Als poröse Träger sind alle dem Fachmann bekannten Materialien mit zumindest Mikro- oder Mesoporen geeignet. Beispielhaft seien hier nur genannt: Aluminiumoxide, aktivierte Aluminiumoxide, Silikate, Kieselsäure, Kieselgur, Wasserglas, Tone, Korunde, Metalloxide, Zeolithe oder Mischungen aus mindestens zwei der vorstehend genannten Substanzen. Die Zusammensetzung Ag mit einem Alkalimetall auf α-Aluminiumoxid ist dabei besonders bevorzugt. Weiter ist ein Verhältnis Ag/Alkalimetall von 1000:1 bis 5000:1 besonders bevorzugt. Eine Ag-Beladung auf dem Trägermaterial von 1 Gew.% bis 5 Gew.-% ist gleichfalls besonders bevorzugt. Die Teil-Schüttung kann neben dem Katalysator noch weitere Zusatz- oder Hilfsstoffe enthalten.

Die zum Schritt (3) gehörige Teil-Schüttung (III), die mindestens ein katalytisch aktives Material enthält, wird in der Figur 2 summarisch als "Li₃PO₄/Steatit" bezeichnet. Dabei kann als katalytisch aktives Material für diese Teil-Schüttung im Prinzip jeder dem Fachmann bekannte Katalysator eingesetzt werden, der die Ringöffnung befördert. Alkalimetall-Phosphate sind hierbei bevorzugt und Li₃PO₄ ist dabei besonders bevorzugt. Als Träger kann im Prinzip jedes Schichtsilikat eingesetzt werden, wobei Talk in jeder Modifikation (Speckstein, Steatit) besonders bevorzugt ist.

Zwischen den Katalysatoren und optional auch vor und/oder nach der ersten/ letzten aktiven Schüttung kann sich optional auch eine nicht notwendigerweise katalytisch aktive Zwischen-Schüttung aus porösem Material befinden, welche im Prinzip zwei Randbedingungen genügen muss: (i) das Material muss genügend gasdurchlässig sein und es sollte (ii) einem Rückvermischen von Edukten und/oder Produkten entgegenwirken. In diesem Kontext sind im Prinzip alle dem Fachmann bekannten und bereits oben genannten porösen Materialien denkbar. Das Verwenden von Korund ist im Rahmen der vorliegenden Erfindung besonders bevorzugt.

Die mindestens eine nicht notwendigerweise katalytisch aktive Zwischen-Schüttung befindet sich in mindestens einer Position innerhalb der strukturierten Katalysator-Schüttung, wobei die besagte Position ausgewählt wird aus der folgenden Gruppe: (i) vor der ersten katalytisch aktiven Teil-Schüttung in Richtung des Edukt-Gasstromes, (ii) zwischen mindestens einem Paar von katalytisch aktiven Teil-Schüttungen sowie (iii) nach der letzten katalytisch aktiven Teil-Schüttung in Richtung des Edukt-Gasstromes.

Es ist weiterhin denkbar, dass sich zwischen und/oder vor und/oder nach einer katalytisch aktiven Teilschicht ein freier Gasraum befindet.

In einer bevorzugten Ausführungsform werden diese verschiedenen Teil-Schüttungen in einem Rohr- und/oder einem Festbettreaktor, wie in Figur 2 schematisch gezeigt, konsekutiv aufeinander geladen. Die Dicke der einzelnen Schichten ist dabei so zu bemessen, dass ein stationärer Betrieb begünstigt wird, d.h. dass kontinuierlich ein Edukt-Gasstrom (=feed) aufgegeben werden kann und dann am anderen Ende der Strom, der das Reaktionsprodukt, hier Crotonaldehyd, enthält, abgezweigt werden kann. Prinzipiell sind der Dicke und/oder Anordnung der einzelnen Schüttungen keine Grenzen gesetzt, solange erfindungsgemäß eine kontinuierliche Umsetzung der Edukte zu Crotonaldehyd möglich ist, und die Schüttung als solche strukturiert ist. Eine Schüttung ist genau dann "strukturiert", wenn zumindest eine Teil-Schüttung erkennbar oder messbar räumlich vom Rest der gesamten Schüttung abgetrennt ist. Die Dicke einer jeden Schicht und die Gesamtdicke sind insbesondere mit der Raumgeschwindigkeit des Gases, bezogen auf eine Stunde (GHSV = gas hourly space velocity; feed Gas Volumen pro Liter an Katalysator und pro Stunde), zu korrelieren, sowie mit dem erwünschten bzw. noch tolerierbaren Druckverlust.

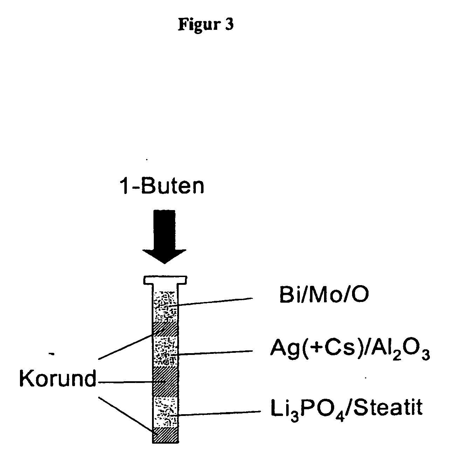

In Figur 3 ist schließlich eine entsprechende dreifache Katalysator-Schüttung dargestellt, wie sie zu verwenden ist, wenn Buten als Edukt verwendet werden soll. Bezüglich der zu den Schritten (2) und (3) gehörigen Teil-Schüttungen sowie den Zwischenschüttungen gilt das oben Geschriebene. Für das Umsetzen von Buten zu Butadien, d.h. für Schritt (1), ist bezüglich der strukturierten Katalysator-Schüttung nun dem Schritt (2) eine weitere Teil-Katalysator-Schüttung (I) vorzuschalten. Diese Teil-Schüttung enthält mindestens ein katalytisch aktives Material, welches zumindest ein Gemisch von mindestens zwei Oxiden der Haupt- und Nebengruppenmetalle enthält. Bevorzugt ist dabei ein Mehrphasen-System mindestens enthaltend Molybdän-Oxid, Wismut-Oxid und Wolfram-Oxid in beliebigen Zusammensetzungen. In diesem Zusammenhang ist der diesbezügliche Inhalt der EP 0 319 754 vollumfänglich in den vorliegenden Text einzubeziehen. In einer besonders bevorzugten Ausführungsform wird ein Oxid enthaltend Mo, Bi, W, Co, Fe, Si und K eingesetzt, wobei die Zusammensetzung Mo₁₂Bi_{1,2}W_{2,4}Co_{4,8}Fe_{0,8}Si_{1,6}K_{0,05}Oₓ besonders bevorzugt ist.

Bezüglich der Reihenfolge der Teil-Schüttungen gilt, dass die katalytisch aktiven Teil-Schüttungen (I), (II) und (III), sowie optional mindestens eine nicht notwendigerweise katalytisch aktive Zwischenschicht prinzipiell in beliebiger Permutation und Wiederholdung oder in beliebiger Permutation oder Wiederholung aufgegeben werden können. Weiterhin gilt, dass diese Teil-Schüttungen in einer bevorzugten Ausführungsform in der Strömungsrichtung des Eduktgases in der Reihenfolge Teil-Schüttung (III) nach Teil-Schüttung (II) oder Teil-Schüttung (III) nach Teil-Schüttung (II) nach Teil-Schüttung (I) aufgegeben werden.

Es ist weiterhin bevorzugt, dass sich die Teilschüttungen zwar in einem gemeinsamen (Rohr)reaktor befinden, dass aber Verfahrensparameter, wie insbesondere Temperatur und/oder Druck und/oder Druckabfall etc. für jede Teilschüttung individuell reguliert werden können. Im Falle der Temperatur kann dies beispielsweise durch verschiedene Heizwicklungen um den Rohrreaktor realisiert werden.

Die beispielhafte Ausbeute und Selektivität wie sie mit der erfindungsgemäßen Katalysator-Schüttung erreicht wird, kann mit den entsprechenden Werten, wie sie nach dem Stand der Technik erhältlich sind, verglichen werden. So offenbart beispielsweise die US 4 942 263 Ergebnisse für die Herstellung von Crotonaldehyd aus 1,3-Butadien. Bei einer Reaktionstemperatur von 225°C und einer GHSV von 2400 h⁻¹ konnten bei Umsätzen von 0,03% Selektivitäten zu Crotonaldehyd von 60% bei einer Feedzusammensetzung von 50% Butadien in O₂ erreicht werden (Ausbeute: 0,018%).

Die angegebenen Werte erscheinen jedoch mit großen Unsicherheiten behaftet, da bei den in Rede stehenden geringen Umsätzen der Fehlerbereich für die Angabe der Selektivitäten sehr hoch wird. In der Druckschrift wird aus den Daten geschlossen, dass die hohe Oberfläche der Trägermaterials hohe Selektivitäten zu Crotonaldehyd zur Folge hat. Diese Schlussfolgerung scheint durch die Daten nicht ausreichend unterstützt. Unabhängig hiervon sind die in dieser Druckschrift beschriebenen Ausbeuten zu Crotonaldehyd (bzw. die entsprechenden Umsätze) deutlich schlechter als die im Falle vorliegenden Erfindung gefundenen Ausbeuten (siehe Ausführungsbeispiele).

Analog kann auch für die oben diskutierte Veröffentlichung von Popova et al. festgestellt werden, dass die Ausbeuten an Crotonaldehyd gering sind, insbesondere im Vergleich zu dem unten diskutierten Beispiel 3 betreffend die erfindungsgemäße Katalysator-Schüttung. Bei Popova et al. wird die Herstellung von Crotonaldehyd aus 1-Buten mit auf Cu basierenden Katalysatoren diskutiert. Bei 370°C und einer GHSV von 8000 h⁻¹ wird bei einer Zusammensetzung des Gasstroms von 4:1:5 (Buten:O₂:N₂) bei einem Umsatz von 1,5% eine Selektivität bezüglich Crotonaldehyd von 16% erreicht (Ausbeute: 0,24%). Als Katalysator wurde 0,5% Cu auf SiO₂ genutzt. Hauptprodukt war hier Methyl-Vinyl-Keton (50-60% Selektivität) und das für die vorliegende Erfindung relevante Produkt Crotonaldehyd ist dabei nur Nebenprodukt.

Im Folgenden soll die Erfindung anhand ausgewählter Ausführungsbeispiele illustriert werden. Dies geschieht ausdrücklich ohne Einschränkung der allgemeinen Gültigkeit des Gegenstandes der vorliegenden Erfindung.

### Beispiel 1: Umsetzung von 1,3-Butadien zu Crotonaldehyd unter Verwendung der erfindungsgemäßen zweifachen Katalysator-Schüttung

Für die Reaktion von 1,3-Butadien zu Crotonaldehyd wurde die erfindungsgemä-ße strukturierte Schüttung aus 1.) Ag(+Cs)/α-Al₂O₃ und 2.) Li₃PO₄/Steatit verwendet. Das Steatit liegt als Granulat mit einer Teilchengröße von 2 bis 3 mm vor. Die Schüttung wurde, wie in allen anderen hier genannten Beispielen auch, in einen Rohrreaktor gegeben (8mm Durchmesser) Dabei wurde unter Zugrundelegung der dem Fachmann bekannten Formeln für Selektivität, Ausbeute und Umsatz und unter Verwendung der Analysedaten aus einem Hewlett Packard HP 5 Gaschromatographen bei 64,2% Umsatz eine Selektivität für Crotonaldehyd von 29,3% bei 250°C, einer Raumgeschwindigkeit des Gases GHSV = 6000 h⁻¹ und einem Einsatz von 1% Butadien/Luft erreicht (Ausbeute: 18,8%). Diesem Ergebnis liegt eine zweifach strukturierte Katalysatorschüttung zugrunde. Es wurden dabei in einem Festbettreaktor Li₃PO₄, Korund und Ag/Al₂O₃ im Volumenverhältnis 1:1:1 übereinandergeschichtet angeordnet (siehe Figur 2).

Die erste Stufe der Schüttung besteht dabei aus einem Katalysator mit 2,5% Ag, 0,001% Cs auf einem α-Al₂O₃-Träger. Die Silber-Fällung wurde hierbei, wie unten beschrieben, mit Ag₂O, Ethanolamin und Ethylendiamin durchgeführt. Die Cs-Quelle war CsCl, das Al₂O₃ stammt von der Firma Ceramtec (Nr. 80411). Die zweite Stufe der Schüttung besteht aus 25% Li₃PO₄ (Aldrich) mit Binder (Kaolin) auf Steatit (Ceramtec, angelaugt). Die Herstellung dieses Katalysators ist gleichfalls unten beschrieben.

### Herstellung von Ag/Al₂O₃ (analog zu US 4 356 312)

Ag wurde auf den Al₂O₃-Träger als Aminkomplex aufgetränkt. Zur Herstellung des Ag-Aminkomplexes wurden 25,2 g H₂C₂O₄ x 2 H₂O, 24 g Ethylendiamin und 46,35 g Ag₂O nacheinander in 100 ml H₂O gelöst. 6,8 g Ethanolamin wurde als Reduktions-/Löslichkeitsagens zugegeben. Diese Lösung wurde mit CsCl gemischt, so dass ein Ag/Cs Verhältnis von 2500:1 entstand. Auf einem α-Al₂O₃-Träger (Ceramtec Nr. 80411) wurde die oben beschriebene Lösung dergestalt aufgetränkt, dass eine Ag-Beladung von 2,5 Gew.-% entstand. Sofort nach Abschluss der Präparation wurde die Probe im Luftstrom (1l/min) innerhalb einer Stunde auf 290°C geheizt und bei dieser Temperatur für weitere drei Stunden gehalten.

### Herstellung der Li₃PO₄/Steatit Schüttung

Für die Präparation der Li₃PO₄/Steatit Schüttung wurden zunächst 25 ml HNO₃ (10%ig) vorgelegt, anschließend zuerst Kaolin (Dorfner) und dann Li₃PO₄ (Aldrich) unter Rühren zugewogen. Die Fällungssuspension wurde dann auf 89 g mit NaOH angelaugtem Steatit der Firma Ceramtec aufgetragen. Dabei wurden die Steatit-Kugeln in dünner Schicht in Porzellanschalen vorgelegt und durch einen Rüttler bei der Aufgabe der Metallsalzlösungen bewegt. Dabei wurden 25 Gew.-% Li₃PO₄ auf Steatit aufgebracht. Die Probe wurde schließlich für 16 h bei 80°C getrocknet.

### Beispiel 2: Umsetzung von 1,3-Butadien zu Crotonaldehyd

Für die Reaktion von 1,3-Butadien zu Crotonaldehyd wurde über eine zweifach strukturierte Schüttung aus 1.) Ag/-αAl₂O₃ und 2.) Li₃PO₄/Steatit bei 39,1% Umsatz eine Selektivität für Crotonaldehyd von 35,8% erzielt (Ausbeute: 14,0%). Reaktionsbedingungen hier waren 225°C, eine GHSV von 6000 h⁻¹ und ein feed von 1% Butadien/Luft. Es wurden dabei in einem Festbettreaktor Li₃PO₄, Korund und Ag/Al₂O₃ im Volumenverhältnis 1:1:1 übereinandergeschichtet angeordnet.

Die erste Stufe der Katalysator-Schüttung bestand aus einem Katalysator mit 5% Ag auf α-Al₂O₃-Träger (wie in Beispiel 1 beschrieben). Das Al₂O₃ stammt dabei von der Firma Ceramtec (Nr. 80411). Die zweite Stufe der Katalysator-Schüttung bestand aus 25% Li₃PO₄ (Aldrich) mit Binder (Kaolin) auf Steatit (Ceramtec, angelaugt). Deren Herstellung ist gleichfalls in Beispiel 1 beschrieben.

### Beispiel 3: Umsetzung von 1-Buten zu Crotonaldehyd unter Verwendung der erfindungsgemäßen dreifachen Katalysator-Schüttung

Für diese Reaktion wurde unter Verwendung einer dreifach strukturierten Schüttung umfassend 1.) Mo₁₂Bi_{1,2}W_{2,4}Co_{4,8}Fe_{0,8}Si_{1,6}K_{0,05}Oₓ 2.) Ag(+K)/α-Al₂O₃ sowie 3.) Li₃PO₄/ Steatit ein Umsatz von 59% und eine Selektivität bezüglich Crotonaldehyd von 17,9% bei 300°C, einer GHSV = 6000 h⁻¹ und dem Einsatz von 1 % Buten/Luft erreicht (Ausbeute = 10,6%). Zur Herstellung der Katalysator-Schüttung wurden dabei in einem Festbettreaktor Li₃PO₄, Korund, Ag/Al₂O₃, wiederum Korund und schließlich ein Bi/Mo/O-Katalysator im Volumenverhältnis 1:1:1:0,5:1 übereinandergeschichtet angeordnet.

Der Mo₁₂Bi_{1,2}W_{2,4}Co_{4,8}Fe_{0,8}Si_{1,6}K_{0,05}Oₓ -Katalysator wurde entsprechend des in der EP 319 754 der BASF AG beschriebenen Verfahrens hergestellt. Der Katalysator der zweiten Stufe bestand dabei aus 0,5% Ag, 0,001% K auf einem α-Al₂O₃-Träger. Die Beschreibung der Herstellung dieser Stufe ist in Beispiel 1 gegeben. Als dritte Stufe wurde wiederum 25% Li₃PO₄ (Aldrich) mit Binder (Kaolin) auf Steatit (Ceramtec, angelaugt) eingesetzt und für die Herstellung dieser Stufe wird wiederum auf Beispiel 1 verwiesen.

Eine Zusammenfassung der in den Beispielen 1 bis 3 sowie in der US 4 942 263 ("Kodak") und in der Veröffentlichung von Popova et al. ("Popova"; siehe Diskussion des Standes der Technik) genannten Selektivitäten (bezüglich Crotonaldehyd, CRA) und Umsätze ist in der folgenden Tabelle gegeben:

| | Feed | Katalysator | Temp. [°C] | GHSV [h⁻¹] | Umsatz [%] | Selektivität (CRA)[%] |
|---|---|---|---|---|---|---|
| Bsp. 1 | 1% Butadien/Luft | Ag(+Cs)/Al₂O₃ + Li₃PO₄/Steatit | 250 | 6000 | 64,2 | 29,3 |
| Bsp. 2 | 1% Butadien/Luft | Ag/Al₂O₃ + Li₃PO₄/Steatit | 225 | 6000 | 39,1 | 35,8 |
| Bsp. 3 | 1% 1-Buten/Luft | Mo/Bi/W/Co/Fe/Si/K/O + Ag(+K)/Al₂O₃ + Li₃PO₄/Steatit | 300 | 6000 | 59,0 | 17,9 |
| Kodak | 50% Butadien/O₂ | Ag/Al₂O₃ | 225 | 2400 | 0,03 | 60 |
| Popova | 40% 1-Buten, 10% O₂, 50% N₂ | Cu/SiO₂ | 370 | 8000 | 1,5 | 16 |

## Patentansprüche

1. Strukturierte Katalysator-Schüttung, **dadurch gekennzeichnet, dass** sie mindestens die folgenden Teil-Schüttungen enthält:
(II) mindestens eine katalytisch aktive Teil-Schüttung mindestens enthaltend Silber, ein Alkalimetall sowie ein poröses Trägermaterial, sowie
(III) mindestens eine katalytisch aktive Teil-Schüttung enthaltend mindestens ein Alkalimetall-Phosphat sowie mindestens ein Schichtsilikat.

2. Strukturierte Katalysator-Schüttung nach Anspruch 1, **dadurch gekennzeichnet, dass** zusätzlich noch die folgende Teil-Schüttung Teil der strukturierten Katalysator-Schüttung ist:
(I) mindestens eine katalytisch aktive Teil-Schüttung enthaltend zumindest ein Gemisch von Oxiden der Haupt- und Nebengruppenmetalle.

3. Strukturierte Katalysator-Schüttung nach Anspruch 1 oder 2, **dadurch gekennzeichnet, dass** die Teil-Schüttungen in der Strömungsrichtung des Eduktgases in der Reihenfolge Teil-Schüttung (III) nach Teil-Schüttung (II) oder Teil-Schüttung (III) nach Teil-Schüttung (II) nach Teil-Schüttung (I) aufgegeben werden.

4. Strukturierte Katalysator-Schüttung nach mindestens einem der vorstehenden Ansprüche, **dadurch gekennzeichnet, dass** sie eine nicht notwendigerweise katalytisch aktive Zwischen-Schüttung oder einen gasfreien Zwischenraum enthält, die/der sich in mindestens einer Position innerhalb der strukturierten Katalysator-Schüttung befindet, wobei die besagte Position ausgewählt wird aus der folgenden Gruppe: (i) vor der ersten katalytisch aktiven Teil-Schüttung in Richtung des Edukt-Gasstromes, (ii) zwischen mindestens einem Paar von katalytisch aktiven Teil-Schüttungen sowie (iii) nach der letzten katalytisch aktiven Teil-Schüttung in Richtung des Edukt-Gasstromes.

5. Strukturierte Katalysator-Schüttung nach mindestens einem der vorstehenden Ansprüche, **dadurch gekennzeichnet, dass** die katalytisch aktiven Teil-Schüttungen (I), (II) und (III), sowie optional mindestens eine nicht notwendigerweise katalytisch aktive Zwischenschicht in beliebiger Permutation und Wiederholdung oder in beliebiger Permutation oder Wiederholung aufgegeben werden.

6. Strukturierte Katalysator-Schüttung nach mindestens einem der vorstehenden Ansprüche, **dadurch gekennzeichnet, dass** die katalytisch aktive Teil-Schüttung (II) Ag mit einem Alkalimetall auf α-Aluminiumoxid enthält.

7. Strukturierte Katalysator-Schüttung nach mindestens einem der vorstehenden Ansprüche, **dadurch gekennzeichnet, dass** die katalytisch aktive Teil-Schüttung (III) mindestens ein Alkalimetall-Phosphat und mindestens ein Schichtsilikat enthält

8. Strukturierte Katalysator-Schüttung nach mindestens einem der vorstehenden Ansprüche, **dadurch gekennzeichnet, dass** die katalytisch aktive Teil-Schüttung (I) mindestens ein Gemisch von mindestens zwei Oxiden der Haupt- und Nebengruppenmetalle enthält.

9. Verfahren zur Herstellung von Crotonaldehyd aus C₄- Mono- oder Diolefinen im stationären Betrieb unter Verwendung der strukturierten Katalysator-Schüttung nach mindestens einem der vorstehenden Ansprüche.

10. Verfahren nach Anspruch 9, **dadurch gekennzeichnet, dass** Butadien im Eduktstrom eingesetzt wird und dass die folgenden beiden Reaktionen mit jeweils den Teil-Schüttungen (II) und (III) katalysiert werden:
(2) Epoxidation von Butadien zu Vinyloxiran;
(3) Isomerisierung von Vinyloxiran zu Crotonaldehyd.

11. Verfahren nach Anspruch 10, **dadurch gekennzeichnet, dass** Buten im Eduktstrom eingesetzt wird und dass zusätzlich zu den in Anspruch 10 genannten Reaktionen noch die folgende Reaktion mit der Teil-Schüttung (I) katalysiert wird:
(1) Dehydrierung von Buten zu Butadien.

## Claims

1. A structured catalyst bed which comprises at least the following part beds:
(II) at least one catalytically active part bed comprising at least silver, an alkali metal and a porous support material, and
(III) at least one catalytically active part bed comprising at least one alkali metal phosphate and at least one sheet silicate.

2. A structured catalyst bed as claimed in claim 1 which further comprises the following part bed:
(I) at least one catalytically active part bed comprising at least a mixture of oxides of the main group metals and transition metals.

3. A structured catalyst bed as claimed in claim 1 or 2, wherein the part beds are installed in the order part bed (III) after part bed (II) or part bed (III) after part bed (II) after part bed (I) in the flow direction of the feed gas.

4. A structured catalyst bed as claimed in any of the preceding claims which comprises a not necessarily catalytically active intermediate bed or a gas-free intermediate space which is present in at least one position within the structured catalyst bed, with this position being selected from the following group: (i) before the first catalytically active part bed in the direction of the feed gas flow, (ii) between at least one pair of catalytically active part beds and (iii) after the last catalytically active part bed in the direction of the feed gas flow.

5. A structured catalyst bed as claimed in any of the preceding claims, wherein the catalytically active part beds (I), (II) and (III) and optionally at least one not necessarily catalytically active intermediate bed are installed in any permutation and repetition or in any permutation or repetition.

6. A structured catalyst bed as claimed in any of the preceding claims, wherein the catalytically active part bed (II) comprises Ag together with an alkali metal on α-aluminum oxide.

7. A structured catalyst bed as claimed in any of the preceding claims, wherein the catalytically active part bed (III) comprises at least one alkali metal phosphate and at least one sheet silicate.

8. A structured catalyst bed as claimed in any of the preceding claims, wherein the catalytically active part bed (I) comprises at least one mixture of at least two oxides of the main group metals and transition metals.

9. A process for preparing crotonaldehyde from C₄-monoolefins or -diolefins in steady-state operation using the structured catalyst bed as claimed in any of the preceding claims.

10. A process as claimed in claim 9, wherein butadiene is used in the feed stream and the following two reactions are catalyzed by the part beds (II) and (III), respectively:
(2) epoxidation of butadiene to vinyloxirane;
(3) isomerization of vinyloxirane to crotonaldehyde.

11. A process as claimed in claim 10, wherein butene is used in the feed stream and, in addition to the reactions specified in claim 10, the following reaction is catalyzed by the part bed (I):
(1) dehydrogenation of butene to butadiene.

## Revendications

1. Garnissage structuré de catalyseurs, **caractérisé en ce qu'**il contient au moins les garnissages partiels suivants :
(II) au moins un garnissage partiel catalytiquement actif contenant au moins de l'argent, un métal alcalin, ainsi qu'une matière de support poreuse, ainsi que
(III) au moins un garnissage partiel catalytiquement actif contenant au moins un phosphate de métal alcalin ainsi qu'au moins un silicate lamellaire.

2. Garnissage structuré de catalyseurs suivant la revendication 1, **caractérisé en ce qu'**en supplément, le garnissage partiel suivant est encore une partie du garnissage structuré de catalyseurs :
(I) au moins un garnissage partiel catalytiquement actif contenant au moins un mélange d'oxydes des métaux des groupes principaux et secondaires.

3. Garnissage structuré de catalyseurs suivant la revendication 1 ou 2, **caractérisé en ce que** les garnissages partiels sont, dans le sens d'écoulement du gaz d'émission, chargés suivant la succession du garnissage partiel (III) après le garnissage partiel (II) ou du garnissage partiel (III) après le garnissage partiel (II) après le garnissage partiel (I).

4. Garnissage structuré de catalyseurs suivant au moins l'une des revendications précédentes, **caractérisé en ce qu'**il contient un garnissage intermédiaire non nécessairement catalytiquement actif ou un volume intermédiaire exempt de gaz, qui se trouve dans au moins une position à l'intérieur du garnissage structuré de catalyseurs, la position précitée étant choisie parmi le groupe suivant : (i) avant le premier garnissage partiel catalytiquement actif dans le sens du courant de gaz d'émission, (ii) entre au moins une paire de garnissages partiels catalytiquement actifs, ainsi que (iii) après le dernier garnissage partiel catalytiquement actif dans le sens du courant de gaz d'émission.

5. Garnissage structuré de catalyseurs suivant au moins l'une des revendications précédentes, **caractérisé en ce que** les garnissages partiels catalytiquement actifs (I), (II) et (III), ainsi qu'éventuellement au moins une couche intermédiaire non nécessairement catalytiquement active sont chargés suivant une permutation et une répétition quelconques ou suivant une permutation ou répétition quelconque.

6. Garnissage structuré de catalyseurs suivant au moins l'une des revendications précédentes, **caractérisé en ce que** le garnissage partiel catalytiquement actif (II) contient Ag avec un métal alcalin sur de l'oxyde d'aluminium α.

7. Garnissage structuré de catalyseurs suivant au moins l'une des revendications précédentes, **caractérisé en ce que** le garnissage partiel catalytiquement actif (III) contient au moins un phosphate de métal alcalin et au moins un silicate lamellaire.

8. Garnissage structuré de catalyseurs suivant au moins l'une des revendications précédentes, **caractérisé en ce que** le garnissage partiel catalytiquement actif (I) contient au moins un mélange d'au moins deux oxydes des métaux des groupes principaux et secondaires.

9. Procédé de préparation d'aldéhyde crotonique à partir de monooléfines ou dioléfines en C₄ dans une mise en service stationnaire, par utilisation du garnissage structuré de catalyseurs suivant au moins l'une des revendications précédentes.

10. Procédé suivant la revendication 9, **caractérisé en ce qu'**on met en oeuvre du butadiène dans le courant de produit de sortie et **en ce que** les deux réactions suivantes sont catalysées avec respectivement les garnissages partiels (II) et (III) :
(2) époxydation de butadiène en vinyloxiranne,
(3) isomérisation de vinyloxiranne en aldéhyde crotonique.

11. Procédé suivant la revendication 10, **caractérisé en ce que** du butène est mis en oeuvre dans le courant de produit de sortie et **en ce qu'**en supplément aux réactions citées dans la revendication 10, la réaction suivante est encore catalysée par le garnissage partiel (I) :
(1) déshydrogénation de butène en butadiène.
